# EUROPÄISCHE PATENTSCHRIFT

(11) **EP 3 986 173 B1**
(45) Veröffentlichungstag und Bekanntmachung des Hinweises auf die Patenterteilung: **18.12.2024**
(21) Anmeldenummer: 20733746.0
(22) Anmeldetag: 17.06.2020
(51) Int. Cl.: A24F 40/10, A24F 40/57, A24F 40/42, A24F 40/44, A61M 11/04, A61M 15/06, A61M 16/00

(54) **VERDAMPFERKARTUSCHE SOWIE INHALATOR MIT EINER SOLCHEN VERDAMPFERKARTUSCHE**
VAPORIZER CARTRIDGE AND INHALER COMPRISING SUCH A VAPORIZER CARTRIDGE
CARTOUCHE DE VAPORISATION ET INHALATEUR POURVU D'UNE TELLE CARTOUCHE DE VAPORISATION

(30) Priorität: 20.06.2019 DE 102019116725
(43) Veröffentlichungstag der Anmeldung: 27.04.2022
(73) Patentinhaber: Körber Technologies GmbH, 21033 Hamburg (DE)
(72) Erfinder: BERGMANN, Max, 22303 Hamburg (DE); CORNILS, Lasse, 22605 Hamburg (DE); GIESE, Matthias, Tokyo 104-0053 (JP); HANNEKEN, Christian, 22299 Hamburg (DE); JAKLIN, Jan, 73054 Eislingen (DE); KESSLER, Marc, 22415 Hamburg (DE); KLEINE WÄCHTER, Michael, 23881 Lankau (DE); MÜLLER, Thomas, 20259 Hamburg (DE); ROMMING, Niklas, 24306 Plön (DE); SCHMIDT, Rene, 21244 Buchholz i.d.N. (DE); SCHUSTER, Christof, 20259 Hamburg (DE); WUTTKE, Tobias, 21465 Reinbek (DE)
(74) Vertreter: Stork Bamberger Patentanwälte PartmbB
(86) Internationale Anmeldenummer: PCT/EP2020/066735
(87) Internationale Veröffentlichungsnummer: WO 2020/254388

(56) Entgegenhaltungen:
- EP-A1- 3 417 726
- EP-A2- 3 200 559
- WO-A1-2013/083638
- WO-A1-2018/211035
- DE-A1- 102017 123 870
- GB-A- 2 561 867
- US-A1- 2016 316 819
- US-A1- 2017 367 411

## Beschreibung

Die Erfindung betrifft eine Verdampferkartusche als Bestandteil eines Inhalators, umfassend einen Hohlkörper mit einem durchgängigen Strömungskanal sowie einen Vorratstank zum Bevorraten von Flüssigkeit, wobei der Vorratstank mindestens eine Zugangsöffnung zum Strömungskanal aufweist und im Bereich jeder Zugangsöffnung eine sich über die gesamte Zugangsöffnung erstreckende Verdampfereinheit angeordnet ist, die ein Dochtorgan und ein Heizorgan aufweist, wobei die Verdampfereinheit flüssigkeitspermeabel ausgebildet ist, derart, dass Flüssigkeit mindestens initial kapillar aus dem Vorratstank durch die Verdampfereinheit in Richtung des Strömungskanals förderbar ist.

Des Weiteren betrifft die Erfindung einen Inhalator, ausgebildet und eingerichtet zum Inhalieren von mit Wirkstoffen angereichertem Dampf, umfassend einen mindestens eine elektronische Steuereinheit und eine Energiequelle umfassenden Kartuschenträger sowie eine Verdampferkartusche.

Solche Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte in Dampfform und/oder als Aerosole inhalieren zu können. Beim Konsumieren saugt üblicherweise eine Person an einem Mundstück des Inhalators, wodurch in dem Strömungskanal ein Saugdruck entsteht, der einen Luftstrom durch den Strömungskanal erzeugt. Der Luftstrom kann aber auch maschinell z.B. durch eine Pumpe erzeugt werden. In dem Strömungskanal wird dem Luftstrom eine von der Verdampfereinheit erzeugte und bereitgestellte verdampfte Flüssigkeit als Gasphase zugegeben und anschließend bei der Mischung mit dem Luftstrom gezielt rekondensiert, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B. Bestandteile von Glycerin und Propylenglycol auf, ggf. angereichert um Nikotin und/oder nahezu beliebige Geschmacksstoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Die einzelnen Bestandteile der Verdampferkartusche, nämlich der Hohlkörper, der Vorratstank und die Verdampfereinheit, können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, nämlich insbesondere der Hohlkörper und die Verdampfereinheit, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine Energiequelle. Die Energiequelle kann z.B. eine elektrochemische Einwegbatterie oder ein wiederaufladbarer elektrochemischer Akku, z.B. ein Li-Ionen-Akku sein, mittels dem das Heizorgan über die elektrischen Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann als Ansteckteil an den Mehrwegartikel ansteckbar oder als Einsetzteil in den Mehrwegartikel einsetzbar ausgebildet sein. Anstelle einer Steckverbindung sind auch Schraubverbindungen oder andere Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Die zentrale und letztlich die Nutzung (z.B. als E-Zigarette oder als medizinischer Inhalator) bestimmende Komponente ist der Vorratstank als Bestandteil der Verdampferkartusche. Diese beinhaltet in der Regel die von der Person gewählte, gewünschte und/oder benötigte Flüssigkeit bzw. ein Flüssigkeitsgemisch (im Folgenden auch allgemein als Fluid bezeichnet) sowie den den Strömungskanal bildenden Hohlkörper und die Verdampfereinheit. Das Fluid ist in dem Vorratstank der Verdampferkartusche bevorratet. Mittels der flüssigkeitspermeablen Verdampfereinheit wird das Fluid aus dem Vorratstank aufgrund zumindest initial kapillarer Förderung durch das Dochtorgan und das Heizorgan geleitet. Die von der Energiequelle erzeugte Spannung, die an dem Heizorgan angelegt wird, führt zu einem Stromfluss im Heizorgan. Aufgrund des Heizwiderstandes, vorzugsweise des Ohm'schen Widerstands des Heizorgans führt der Stromfluss zu einer Erhitzung des Heizorganes und letztlich zu einer Verdampfung des in der Verdampfereinheit befindlichen Fluids. Das auf diese Weise erzeugte Gas bzw. der Dampf und/oder Aerosol entweicht aus der Verdampfereinheit in Richtung des Strömungskanals und wird durch Mischung mit der Luftströmung gezielt zu einem Aerosol rekondensiert. Das Fluid hat damit einen vorgegebenen Weg mit einer vorgegebenen Strömungsrichtung, nämlich als Fluid durch das Dochtorgan an das und durch das Heizorgan und als Gas/Dampf/Nebel und/oder Aerosol aus dem Heizorgan in den Strömungskanal. In dem Strömungskanal wird das verdampfte Fluid durch den Luftstrom mitgerissen, wenn der Strömungskanal mit einem Druck/Unterdruck beaufschlagt wird, indem z.B. eine konsumierende Person an dem Strömungskanal saugt oder eine Pumpe einen Luftstrom durch den Strömungskanal pustet.

Damit das Fluid aus dem Vorratstank nicht direkt in den Strömungskanal fließt, deckt die Verdampfereinheit den Zugang vom Vorratstank zum Strömungskanal vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Strömungskanal gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Strömungskanals und wirkt gleichzeitig als eine Art Rückschlagschutz, um den Rücklauf von Fluid und/oder Dampf in Richtung des Vorratstanks zu unterbinden.

Bisher bekannte Verdampferkartuschen weisen eine Verdampfereinheit mit einem Dochtorgan auf, das aus mehreren miteinander verwobenen/verdrillten Fäden/Fasern z.B. aus Baumwolle oder aus Glasfasern gebildet ist. Dieser Faserdocht weist kapillare Eigenschaften auf, die dazu führen, dass beim initialen Kontakt mit dem Fluid, der Faserdocht taucht in den Vorratstank ein, das Fluid in dem Vorratstank aufgenommen und in Richtung des Heizorgans gefördert wird. Das Heizorgan ist üblicherweise in Form einer Glühwendel ausgebildet. Dieser gewickelte Metalldraht besteht beispielsweise aus Edelstahl, Kupfer, Kupferverbindungen oder Nickel. Diese Verdampfereinheit ist in der Regel nur manuell herzustellen und weist eine begrenzte Speicherkapazität zum Zwischenspeichern von Fluid auf. Ein weiterer Nachteil besteht in der geringen Transportrate von Fluid aufgrund der begrenzten Anzahl von Mikrokanälen sowie einer systembedingt über die Länge des Docht-Wendel-Systems inhomogenen Temperaturverteilung. Mit anderen Worten ist eine gleichmäßige und kontinuierliche Versorgung des Heizorganes mit dem Fluid nur begrenzt sichergestellt. Des Weiteren weist diese Lösung keinen Rückschlagschutz auf.

Bei anderen bekannten Lösungen umfasst die Verdampfereinheit daher einen einstückigen Dochtblock als Dochtorgan. Dieser üblicherweise aus keramischen Werkstoffen bestehende Dochtblock vereinfacht die automatisierte Herstellung der Verdampfereinheit und der Verdampferkartusche und weist mehrere Mikrokanäle für eine gegenüber dem Faserdocht erhöhte Transportrate auf. Dennoch weist auch diese Lösung mehrere Nachteile auf. Neben einer weiterhin limitierten Transportrate und Zwischenspeicherkapazität ist der Einsatz solcher blockartigen Dochtblöcke sehr unflexibel und vor allem schwierig zu montieren, da die Dochtblöcke nur in exakt - in einem engen Toleranzbereich - vorgefertigte Aufnahmen/Halterungen oder dergleichen einsetzbar sind.

Bei einem einstückigen Dochtkörper kann dieser selbst als Heizorgan dienen, wenn das z.B. keramische Material des Dochtkörpers, der Mikrokanäle aufweist, elektrisch leitfähig ausgebildet ist. Dann weist der Dochtkörper eine Doppelfunktion auf und bildet die Verdampfereinheit. In anderen Fällen kann zusätzlich zum Dochtkörper eine separate Komponente als Heizorgan dienen. In dem letztgenannten Fall bilden der Dochtkörper und das separate Heizorgan die Verdampfereinheit. Das Heizorgan kann wenigstens ein Halbleitermaterial und/oder ein dotiertes Halbleitermaterial, vorzugsweise ein p- oder n-dotiertes Halbleitermaterial, beispielsweise mono- oder polykristallines Silizium und/oder mono- oder polykristallines p- oder n-dotiertes Silizium aufweisen. Das Heizorgan kann aber auch aus Silizium bestehen. Das Heizorgan ist dann üblicherweise ein flächiges und flaches MEMS-Bauteil (Microelectro-mechanical-system-Bauteil), das flüssigkeitspermeabel ausgebildet ist. In beiden Fällen ist der Dochtkörper insbesondere durch Klemmung am Hohlkörper bzw. zwischen Hohlkörper und anderen Komponenten der Verdampferkartusche eingeklemmt. Insbesondere ist eine reproduzierbare Befestigung des Dochtkörpers mittels Presspassung aufgrund von Fertigungstoleranzen schwierig.

Erfindungsgemäß soll zur Überwindung der vorgenannten Nachteile anstelle des Faserdochts bzw. des einstückigen Dochtblocks als Dochtkörper ein Dochtorgan eingesetzt werden, das aus einer Vielzahl von granulatartigen und losen Körnern gebildet ist. Ein solches granulares Dochtorgan lässt sich nicht ohne Weiteres mit herkömmlichen Mitteln in Position halten, insbesondere nicht einklemmen.

Die Erfindung ist in Anspruch 1 definiert. Bevorzugte Ausführungen der Erfindung sind in den abhängigen Ansprüchen beschrieben.

Der Erfindung liegt somit die Aufgabe zugrunde, eine die genannten Nachteile überwindende Verdampferkartusche mit einem granularen Dochtorgan gemäß Anspruch 1 vorzuschlagen, das auf einfache und sichere Weise in seiner Position gehalten wird. Die Aufgabe besteht weiterhin darin, einen entsprechenden Inhalator vorzuschlagen.

Diese Aufgabe wird durch eine Verdampferkartusche der eingangs genannten Art dadurch gelöst, dass das Dochtorgan aus einer Vielzahl granulatartiger Körner gebildet ist, die aufgrund ihrer Schüttung und/oder Ausbildung Mikrokanäle bilden, die sich zur Herstellung einer Fluidverbindung zwischen dem Vorratstank und dem Strömungskanal durchgängig von einer Eintrittsseite E_{D} des Dochtorgans zu einer Austrittsseite A_{D} des Dochtorgans erstrecken, wobei ein zusätzliches Befestigungsmittel vorgesehen ist, das ausgebildet und eingerichtet ist, das granulare Dochtorgan in einer die Fluidverbindung aufrechterhaltenden Weise im Bereich der Zugangsöffnung in Position zu halten. Mit Schüttung ist sowohl das lose als auch das verbundene Nebeneinanderliegen der Körner beschrieben, wobei auch gerüttelte und/oder verdichtete Anordnungen der Körner umfasst sind. Mit Ausbildung der Körner ist z.B. beschrieben, dass die Körner selbst Mikrohohlräume und/oder Mikrokanäle aufweisen können. Die Mikrokanäle bilden sich durch die aneinander liegenden Körner. Somit werden in der Verdampfereinheit zwischen den einzelnen Körnern und/oder durch einzelne Körner eine Vielzahl zufälliger Mikrokanäle zwischen dem Vorratstank und dem Strömungskanal gebildet, die eine konstante und gleichmäßige Verdampfung auf der Austrittsseite der Verdampfereinheit sicherstellen. Anders ausgedrückt wird durch das granulare Dochtorgan eine optimale Fluidkopplung zwischen der Eintrittsseite in die Verdampfereinheit und der Austrittsseite aus der Verdampfereinheit hergestellt. Mit dem zusätzlichen "Befestigungsmittel" ist zunächst übergeordnet alles beschrieben, was die Körner als Dochtorgan in Position halten kann, also u.a. mechanische, physikalische, chemische, magnetische oder elektrostatische Befestigungsoptionen oder Kombinationen davon.

Mit der erfindungsgemäßen Ausbildung des granularen Dochtorgans werden gegenüber den aus dem Stand der Technik bekannten Lösungen eine Fülle von Vorteilen erzielt. Neben der verbesserten Transportrate, es wird mehr und vor allem gleichmäßig Fluid aus dem Vorratstank durch die Verdampfereinheit geführt, gewährleistet das granulare Dochtorgan aufgrund der körnigen und somit porösen Dochtstruktur eine erhöhte Zwischenspeicherkapazität für Fluid. Des Weiteren verbessert ein granulares Dochtorgan den Rückschlagschutz, da die gebildeten Mikrokanäle einen nichtlinearen Verlauf aufweisen. Besonders vorteilhaft gestaltet sich die Montage eines granularen Dochtorgans, da sich dieses an dem jeweiligen Montageort an jede beliebige Kontur/Geometrie der Aufnahme des Dochtorgans anpassen kann. Durch die Kornstruktur passt sich das Dochtorgan beim Montieren/Abfüllen des granularen Materials flexibel an die jeweilige Kontur/Geometrie an und füllt die durch die jeweilige Kontur/Geometrie gebildeten - nicht Mikrokanäle bildende - Hohlräume auf und vermeidet Spaltbildung zu angrenzenden Flächen. Im Ergebnis werden durch das granulare Dochtorgan konstante und reproduzierbare Verdampfungsbedingungen sicherstellt. Dabei spielt es keine Rolle, ob die Verdampfereinheit - mit dem Dochtorgan und/oder dem Heizorgan als Bestandteil der Verdampferkartusche - an dem oder in dem Kartuschenträger angeordnet ist, also am/im Mehrwegartikel, oder ob die Verdampfereinheit am/im Einwegartikel angeordnet ist. Das zusätzliche Befestigungsmittel ermöglicht zum einen eine automatisierte Montage/Herstellung der Verdampferkartusche, also insbesondere das automatische Positionieren des Dochtorgans im Bereich der Zugangsöffnung, und zum anderen das Halten der Position des granularen Dochtorgans im Bereich der Zugangsöffnung.

Eine bevorzugte Ausführungsform ist dadurch gekennzeichnet, dass das Befestigungsmittel mechanischer Art ist, derart, dass aus Komponenten der Verdampferkartusche oder Teilen davon ein allseitig umschlossener Aufnahmeraum mit einer Öffnung zum Einbringen des granularen Dochtorgans gebildet und die Öffnung mit einem Abdeckelement verschlossen ist, wobei der Aufnahmeraum mindestens abschnittsweise flüssigkeitspermeabel ausgebildet ist. Die granularen Körner füllen den Aufnahmeraum maximal aus, vorzugsweise ohne unerwünschte - nicht Mikrokanäle bildende - Hohlräume und/oder Spalte, so dass das Dochtorgan unabhängig von Fertigungstoleranzen an nahezu jede beliebige Form der Umgebung anpassbar ist. Die flüssigkeitspermeablen Abschnitte des Aufnahmeraums sind insbesondere in Richtung Vorratstank und in Richtung Strömungskanal ausgebildet. Die Öffnung im Aufnahmeraum ermöglicht das automatisierte Einbringen/Einfüllen des Dochtorgans in den Aufnahmeraum. Mit dem Abdeckelement wird das granulare Dochtorgan in seiner finalen Position gehalten. Das Abdeckelement selbst kann auch flüssigkeitspermeabel ausgebildet sein.

Vorteilhafterweise umfasst die Verdampferkartusche ein Trägerelement, das den Hohlkörper bildet und zum einen eine Durchgangsöffnung zur Bildung des Strömungskanals aufweist und zum anderen eine Ausnehmung zur Aufnahme des Dochtorgans aufweist, wobei die Ausnehmung die Seitenwände des Aufnahmeraums bildet und die Ausnehmung zum Vorratstank hin und zum Strömungskanal hin jeweils durch eine flüssigkeitspermeable Struktur begrenzt ist. Die Struktur, z.B. eine Gitterstruktur, zum Vorratstank hin ist vorzugsweise das Abdeckelement._Jede Struktur kann aus Metall, Kunststoff, Vlies oder anderen flexiblen und nicht-flexiblen Materialien oder Materialmischungen gebildet sein. Das Trägerelement selbst bildet somit einen Teil des Befestigungsmittels, das durch (Gitter-)Strukturen, Heizorgane oder andere flüssigkeitspermeable Komponenten komplettiert wird. Das eigentliche Rückhalteelement des Dochtorgans, das diesen in der Ausnehmung hält, ist das Abdeckelement, das mit dem Trägerelement verbunden ist. Der Aufnahmeraum als Bestandteil des Befestigungsmittels hält das Dochtorgan und das Heizorgan für den Fall, dass ein separates Heizorgan zum Dochtorgan vorgesehen ist, zusammen und in Position, und zwar vor der Zugangsöffnung zwischen Vorratstank und Strömungskanal, so dass die Flüssigkeit einerseits zuverlässig darin gehindert wird, direkt aus dem Vorratstank in den Strömungskanal zu fließen, und andererseits gleichmäßig und konstant in Richtung Strömungskanal geführt wird.

Eine zweckmäßige Weiterbildung ist dadurch gekennzeichnet, dass die Körner des granularen Dochtorgans durch ein Sieb in Position gehalten werden, derart, dass das Sieb zusammen mit den den Aufnahmeraum begrenzenden Seitenwänden und einem separat zum Dochtorgan ausgebildeten Heizorgan das Volumen des Dochtorgans umschließt. Diese Ausführungsform ermöglicht das einfache und automatisierte Einbringen des Dochtorgans in den Aufnahmeraum sowie das in Position Halten des Dochtorgans im Aufnahmeraum. Insbesondere kann das Sieb als Rückhalteelement einerseits in wenigstens einen Teil der Körner eingreifen und andererseits die Fluidverbindung zwischen dem Vorratstank und dem Dochtorgan aufrechterhalten.

Vorzugsweise ist das Abdeckelement wahlweise ein fester oder weicher Deckel. Als fester Deckel ist zum Beispiel ein aus Metall gebildetes Sieb einsetzbar. Ein weicher Deckel kann z.B. ein flüssigkeitspermeabler Silikonstopfen oder ein einfacher Vliesstreifen sein.

In einer bevorzugten Weiterbildung deckt der Deckel mindestens die Öffnung ab, vorzugsweise umgibt der Deckel jedoch den gesamten Hohlkörper einschließlich der Öffnung des Aufnahmeraums, wobei der Deckel mindestens abschnittsweise flüssigkeitspermeabel ausgebildet ist. Mit diesem Deckel sind eine einfache und sichere Positionierung und das Halten des Dochtorgans in der Position gewährleistet.

In einer weiteren bevorzugten Ausführungsform ist der Deckel eine gegenüber dem Hohlkörper längsaxial oder rotatorisch verschiebbare Hülse, die mindestens abschnittsweise flüssigkeitspermeabel ausgebildet ist. Mit der Hülse sind eine einfache und sichere Positionierung und das Halten des Dochtorgans in der Position gewährleistet.

In einer weiteren bevorzugten Weiterbildung weist der Deckel einen gewickelten Vliesstreifen auf oder ist der Deckel aus einem gewickelten Vliesstreifen gebildet. Der Vliesstreifen als Rückhalteelement ist besonders leicht und hält das Dochtorgan, bei optimaler Flüssigkeitspermeabilität, in Position.

In einer weiteren bevorzugten Ausführungsform ist das Befestigungsmittel physikalischer und/oder chemischer Art, derart, dass die Körner durch Schmelz- und/oder Aushärteprozesse in Position gehalten werden. Diese Art Befestigungsmittel kann mit mechanischen Befestigungsmitteln oder Teilen davon kombiniert sein. Durch das Verschmelzen der Körner untereinander und/oder mit benachbarten Strukturen, beispielsweise dem Trägerelement, lässt sich ein sicheres Rückhaltemittel für das Dochtorgan bilden.

Vorteilhafterweise sind Körner des granularen Dochtorgans direkt mit dem Hohlkörper bzw. dem Trägerelement verschmolzen. Damit ist eine einfache und sichere Fixierung des granularen Dochtorgans gewährleistet.

Eine zweckmäßige Weiterbildung sieht vor, dass verschmolzene Körner des granularen Dochtorgans ihren eigenen geschlossenen und mindestens abschnittsweise flüssigkeitspermeablen Aufnahmeraum bilden. Damit ist ebenfalls eine einfache und sichere Fixierung des granularen Dochtorgans gewährleistet.

Vorzugsweise ist mindestens das Abdeckelement aus verschmolzenen Körnern aus wenigstens einem Teil des granularen Dochtorgans gebildet. Mit dieser Ausbildung bildet das Dochtorgan selbst oder Teile davon sein Rückhaltemittel, um das Dochtorgan in Position zu halten.

In einer weiteren bevorzugten Ausführungsform ist das Befestigungsmittel magnetischer Art, derart, dass die Körner mindestens teilweise magnetisch ausgebildet sind und Magnete, die im Bereich des Dochtorgans angeordnet sind, die Körner in Position halten.

In einer weiteren bevorzugten Ausführungsform ist das Befestigungsmittel elektrostatischer Art, derart, dass die Körner mindestens teilweise elektrisch geladen sind und elektrische Felder, die im Bereich des Dochtorgans vorhanden sind, die Körner in Position halten.

Vorzugsweise sind die Befestigungsmittel wahlweise für eine dauerhafte oder eine temporäre Befestigung ausgebildet und eingerichtet. Als Beispiel eines dauerhaften Befestigungsmittels ist ein aufsteckbarer Deckel genannt, der im aufgesteckten Zustand verhindert, dass lose in den Aufnahmeraum geschüttete Körner aus dem Aufnahmeraum fallen. Mit anderen Worten hält der Deckel die Körner in Position. Als Beispiel eines temporären Befestigungsmittels ist das Dochtorgan als Block mindestens zu Montagezwecken der Verdampferkartusche aus einer gefrorenen oder schockgefrosteten Flüssigkeit-Granulat-Paste gebildet. Dieser gefrorene oder schockgefrostete Block hält die Körner für die Zeit der Montage zusammen. Dieser Block lässt sich automatisiert in den Aufnahmeraum einbringen. Mit einem Deckel ist der Aufnahmeraum verschließbar, so dass die nach dem Auftauen losen Körner des Dochtorgans in Position gehalten werden.

Die Aufgabe wird auch durch einen Inhalator der eingangs gennannten Art dadurch gelöst, dass die Verdampferkartusche nach einem oder mehreren der Ansprüche 1 bis 15 ausgebildet und eingerichtet ist.

Die sich daraus ergebenden Vorteile wurden bereits im Zusammenhang mit der Verdampferkartusche beschrieben, weshalb zur Vermeidung von Wiederholungen auf die vorstehenden Ausführungen verwiesen wird.

Weitere zweckmäßige und/oder vorteilhafte Merkmale und Weiterbildungen zur Verdampferkartusche und dem Inhalator ergeben sich aus den Unteransprüchen und der Beschreibung. Besonders bevorzugte Ausführungsformen der Verdampferkartusche und des Inhalators werden anhand der beigefügten Zeichnung näher erläutert. In der Zeichnung zeigt:
- Fig. 1: eine schematische Darstellung einer bevorzugten Ausführungsform eines erfindungsgemäßen Inhalators mit Kartuschenträger und Verdampferkartusche im Teilschnitt,
- Fig. 2: eine vergrößerte Darstellung der Verdampferkartusche gemäß Figur 1 im Teilschnitt,
- Fig. 3: eine vergrößerte Darstellung einer bevorzugten Ausführungsform eines Teils einer Verdampferkartusche im Schnitt,
- Fig. 4: eine weitere Ausführungsform eines Teils einer Verdampferkartusche,
- Fig. 5: eine weitere Ausführungsform eines Teils einer Verdampferkartusche,
- Fig. 6: eine vergrößerte Darstellung einer weiteren Ausführungsform eines Teils einer Verdampferkartusche,
- Fig. 7: die Verdampferkartusche gemäß Figur 6 entlang des Schnitts A-A,
- Fig. 8: eine vergrößerte Darstellung einer weiteren Ausführungsform einer Verdampferkartusche im Teilschnitt,
- Fig. 9: die Verdampferkartusche gemäß Figur 8,
- Fig. 10: eine vergrößerte Darstellung einer weiteren Ausführungsform einer Verdampferkartusche im Teilschnitt, und
- Fig. 11: die Verdampferkartusche gemäß Figur 10.

Die in der Zeichnung dargestellte Verdampferkartusche sowie der Inhalator dienen zum Inhalieren von mit Wirkstoffen, z.B. Nikotin, angereichertem Dampf und/oder Aerosolen aus Flüssigkeiten, sind entsprechend im Zusammenhang mit einer E-Zigarette beschrieben. Die Verdampferkartusche und der Inhalator sind in gleicher Weise zum Inhalieren von mit medizinischen Wirkstoffen angereichertem Dampf aus pharmazeutischen und/oder nahrungsergänzenden Produkten einsetzbar.

Die dargestellte Verdampferkartusche 10 umfasst einen Hohlkörper 15 mit einem durchgängigen Strömungskanal 16 sowie einen Vorratstank 17 zum Bevorraten von Flüssigkeit, wobei der Vorratstank 17 mindestens eine Zugangsöffnung 18 zum Strömungskanal 16 aufweist und im Bereich jeder Zugangsöffnung 18 eine sich über die gesamte Zugangsöffnung 18 erstreckende Verdampfereinheit 19 angeordnet ist, die ein Dochtorgan 20 und ein Heizorgan 21 aufweist, wobei die Verdampfereinheit 19 flüssigkeitspermeabel ausgebildet ist, derart, dass die Flüssigkeit mindestens initial kapillar aus dem Vorratstank 17 durch die Verdampfereinheit 19 in Richtung des Strömungskanals 16 förderbar ist.

Der Hohlkörper 15 mit seinem mindestens einen Strömungskanal 16 bildet einen Saugkanal/Schlot. Die Form des Hohlkörpers 15 kann ebenso wie der Verlauf jedes Strömungskanals 16 nahezu beliebig sein. Entscheidend ist, dass die Eintrittsseite Es jedes Strömungskanals 16 offen zur Umgebung ist, um z.B. Luft ansaugen zu können, und dass die Austrittsseite As offen ist, um ein z.B. einen Unterdruck anlegen zu können, insbesondere durch das Saugen einer konsumierenden Person. Offen bedeutet in diesem Zusammenhang, dass die Eintrittsseite Es und die Austrittsseite As luftdurchlässig sind. Es können auch zwei oder mehr Strömungskanäle 16 mit entsprechenden Eintrittsseiten Es und Austrittsseiten As vorgesehen sein. Im Bereich der Zugangsöffnung 18 zwischen Vorratstank 17 und Strömungskanal 16 bildet die Verdampfereinheit 19 eine Art Flüssigkeitssperre, die verhindert, dass Flüssigkeit aus dem Vorratstank 17 direkt und als Flüssigkeit in den Strömungskanal 16 fließt. Unabhängig von der Form und Ausbildung des Vorratstanks 17, es können auch zwei oder mehr Vorratstanks 17 vorgesehen sein, und des Hohlkörpers 15 sowie der Anordnung/Positionierung von Vorratstank 17 zu Hohlkörper 15 stellt die Verdampfereinheit 19 sicher, dass Flüssigkeit zwangsläufig aus dem Vorratstank 17 in Richtung des Strömungskanals 16 geführt wird und spätestens beim Austritt aus der Verdampfereinheit 19 als Dampf in den oder jeden Strömungskanal 16 abgegeben wird.

Diese Verdampferkartusche 10 zeichnet sich erfindungsgemäß dadurch aus, dass das Dochtorgan 20 aus einer Vielzahl granulatartiger Körner 24 gebildet ist, die aufgrund ihrer Schüttung und/oder Ausbildung Mikrokanäle 23 bilden, die sich zur Herstellung einer Fluidverbindung zwischen dem Vorratstank 17 und dem Strömungskanal 16 durchgängig von einer Eintrittsseite E_{D} des Dochtorgans 20 zu einer Austrittsseite A_{D} des Dochtorgans 20 erstrecken, wobei ein zusätzliches Befestigungsmittel 25 vorgesehen ist, das ausgebildet und eingerichtet ist, das granulare Dochtorgan 20 in einer die Fluidverbindung aufrechterhaltenden Weise im Bereich der Zugangsöffnung 18 in Position zu halten.

Die Körner 24 bilden zum einen mit benachbarten Körnern 24 (Mikro-)Hohlräume. Zum anderen können die Körner 24 selbst (Mikro-)Hohlräume, so genannte Poren, aufweisen. Durch die Anbindung und das Zusammenwirken sämtlicher Mikrohohlräume in den und/oder zwischen den Körnern 24 sind die Mikrokanäle 23 gebildet, die eine mindestens initial kapillare Förderung gewährleisten sowie durchgängig ausgebildet sind und einen nichtlinearen Verlauf aufweisen. Beim Durchtritt durch die das Dochtorgan 20 und das Heizorgan 21 umfassende Verdampfereinheit 19 wird im Betrieb der Verdampferkartusche 10 aus der Flüssigkeit des Vorratstanks 17 zum Strömungskanal 16 hin Dampf und/oder Aerosol gebildet, wobei die poröse Struktur des Dochtorgans 20 einerseits ein Speichermedium für Flüssigkeit bildet und andererseits einen Strömungswiderstand darstellt. Die Strömungsrichtung der Flüssigkeit erfolgt vom Vorratstank 17 durch die Verdampfereinheit 19 in Richtung des Strömungskanals 16.

Mit dem zusätzlichen "Befestigungsmittel" 25 ist zunächst übergeordnet alles beschrieben, was die Körner 24 mindestens zu Zwecken der Montage, also während des Einbringens des Dochtorgans 20 in den Bereich der Zugangsöffnung 18, vorzugsweise jedoch auch in montiertem Zustand der Verdampferkartusche 10, als Dochtorgan 20 in Position halten kann, also u.a. mechanische, physikalische, chemische, magnetische oder elektrostatische Befestigungsoptionen oder Kombinationen davon.

Die erfindungsgemäße Verdampferkartusche 10 kann als Einwegartikel eine bauliche Einheit sein, die die Komponenten Hohlkörper 15, Vorratstank 17 und Verdampfereinheit 19 beinhaltet. Die Verdampferkartusche 10 kann aber auch mehrteilig ausgebildet sein, wobei sich Komponenten der Verdampferkartusche 10 auf den Einwegartikel und den Mehrwegartikel verteilen, derart, dass z.B. der Vorratstank 17 ein Einwegartikel ist, der erst beim Zusammenführen mit einem Kartuschenträger 13, der ein Mehrwegartikel sein kann und neben einer elektronischen Steuereinheit 11 und einer Energiequelle 12 auch Bestandteile der Verdampferkartusche 10, wie z.B. den Hohlkörper 15 und die Verdampfereinheit 19, umfassen kann, zur baulichen Einheit der Verdampferkartusche 10 führt. Die Verdampferkartusche 10 definiert sich entsprechend über ihre Komponenten, nämlich Hohlkörper 15 mit Strömungskanal 16, Vorratstank 17 und Verdampfereinheit 19, und nicht über die konstruktive/bauliche Zuordnung der Komponenten zum Mehrwegartikel bzw. Einwegartikel.

Die im Folgenden beschriebenen Merkmale und Weiterbildungen stellen für sich betrachtet oder in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig die weiter oben beschriebene Verdampferkartusche 10 weiterbilden können.

Bevorzugt ist die Verdampferkartusche 10 zum mechanischen und elektrischen Verbinden mit dem mindestens die elektronische Steuereinheit 11 und die Energiequelle 12 umfassenden Kartuschenträger 13 zur Bildung eines Inhalators 14 ausgebildet und eingerichtet, wobei die Verdampfereinheit 19 elektrische Kontakte 22 zur elektrischen Kontaktierung mit der Energiequelle 12 umfasst. Der Inhalator 14 kann z.B. durch eine inhalierende Person aktiviert werden, beispielsweise als E-Zigarette, oder z.B. durch eine Pumpe aktiviert werden, z.B. als medizinisches Instrument für den Fall, das die Person selbst nicht mehr oder nicht ausreichend saugen kann.

In einer bevorzugten Ausführungsform ist das Befestigungsmittel 25 mechanischer Art, derart, dass aus Komponenten der Verdampferkartusche 10 oder Teilen davon ein allseitig umschlossener Aufnahmeraum 26 mit einer Öffnung 27 zum Einbringen des granularen Dochtorgans 20 gebildet und die Öffnung 27 mit einem Abdeckelement 28 verschlossen ist, wobei der Aufnahmeraum 26 mindestens abschnittsweise flüssigkeitspermeabel ausgebildet ist. Mit dem Aufnahmeraum 26 ist ein Volumen oder eine Art Käfig gebildet, in das bzw. den das Dochtorgan 20 einbringbar und durch das Abdeckelement 28 in Position haltbar ist. Das Dochtorgan 20 kann als lose Schüttung der Körner 24 in den offenen Aufnahmeraum 26 geschüttet oder als Körnerverbund in den offenen Aufnahmeraum 26 eingesetzt werden, wobei das Dochtorgan 20 in montiertem Zustand der Verdampferkartusche 10 durch das Abdeckelement 28 in Position gehalten wird, und zwar in der Zugangsöffnung 18 zwischen Vorratstank 17 und Strömungskanal 16. Das Befestigungsmittel 25 mechanischer Art umschließt das Dochtorgan 20 vollständig und gewährt gleichzeitig eine Fluidverbindung zum Vorratstank 17 einerseits und in Richtung Strömungskanal 16 andererseits. Das mechanische Befestigungsmittel 25 ist ein Rückhaltemittel, das mit wenigstens einem Teil der Körner des Dochtorgans 20 in Eingriff stehen kann.

Vorzugsweise umfasst die Verdampferkartusche 10 ein Trägerelement 29, das den Hohlkörper 15 bildet und zum einen eine Durchgangsöffnung 30 zur Bildung des Strömungskanals 16 aufweist, und zum anderen eine Ausnehmung 31 zur Aufnahme der Verdampfereinheit 19 aufweist, wobei die Ausnehmung 31 die Seitenwände 32 des Aufnahmeraums 26 bildet und die Ausnehmung 31 zum Vorratstank 17 hin und zum Strömungskanal 16 hin jeweils durch eine flüssigkeitspermeable Struktur begrenzt ist. Die Verdampfereinheit 19 kann alleine durch das Dochtorgan 20 gebildet sein, das für den Fall, das die Körner 24 mindestens teilweise elektrisch leitfähig ausgebildet sind, gleichzeitig das Heizorgan 21 darstellt. Um das Dochtorgan 20 in Position, also in der Ausnehmung 31 zu halten, ist dann z.B. eine Struktur, z.B. eine Gitterstruktur, notwendig. Bevorzugt ist die Verdampfereinheit 19 jedoch zweiteilig ausgebildet, derart, dass Dochtorgan 20 und Heizorgan 21 separate Elemente sind. Im letztgenannten Fall bildet z.B. das Heizorgan 21 die flüssigkeitspermeable Struktur zum Strömungskanal 16 hin.

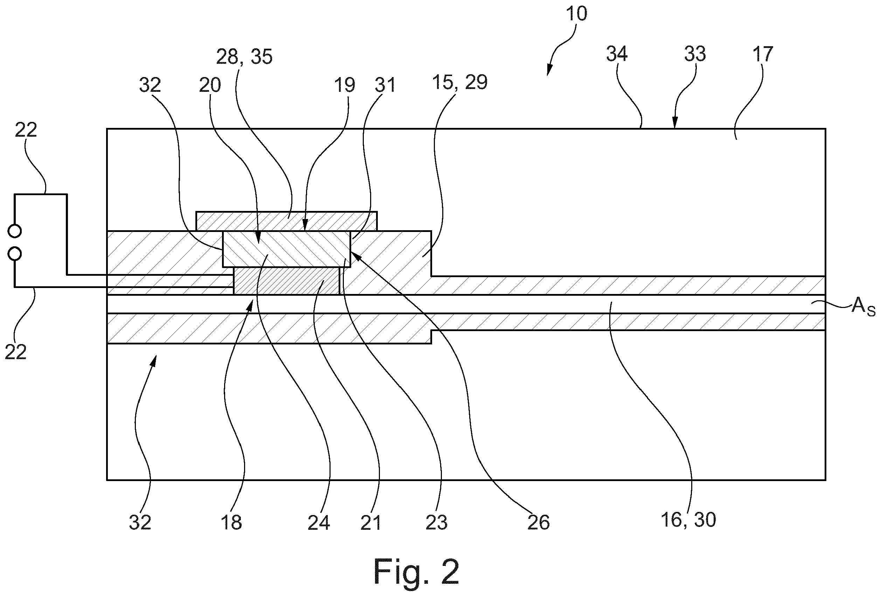

Das Trägerelement 29 ist vorzugsweise ein rohrförmiger Körper. Das Trägerelement 29 kann aber auch andere Formen aufweisen. In der Umfangswand des Körpers ist die Ausnehmung 31 ausgebildet, in der die Verdampfereinheit 19 angeordnet ist. Vorzugsweise ist die aus Trägerelement 29 und Verdampfereinheit 19 gebildete Einheit innerhalb eines den Vorratstank 17 bildenden Gehäuses 33 angeordnet, wobei das Innenvolumen des Vorratstanks 17 zwischen einer Gehäusewand 34 des Gehäuses 33 und dem Trägerelement 29 gebildet ist. Das Trägerelement 29 kann sich nur teilweise durch das Gehäuse 33 erstrecken. In anderen Ausführungsformen kann sich das Trägerelement 29 auch vollständig (siehe z.B. Figur 2) durch das Gehäuse 33 erstrecken.

Die Körner 24 des granularen Dochtorgans 20 werden in einer anderen Ausführungsform durch ein Sieb in Position gehalten, derart, dass das Sieb zusammen mit den den Aufnahmeraum 26 begrenzenden Seitenwänden 32 und einem separat zum Dochtorgan 20 ausgebildeten Heizorgan 21 das Volumen des Dochtorgans 20 umschließt. Einfach ausgedrückt ist der Aufnahmeraum 26 zu den Seiten und nach unten durch die Seitenwände 32 und das Heizorgan 21 begrenzt, um durch die Öffnung 27 das Dochtorgan 20 - vorzugsweise automatisiert - von oben in den Aufnahmeraum 26 zu bringen, zu schütten oder anderweitig einzusetzen. Mittels des Siebs ist der vom Dochtorgan 20 ausgefüllte Aufnahmeraum 26 verschlossen. Das Heizorgan 21 durch flüssigkeitspermeable Durchgänge und das Sieb durch seine gitterartige und/oder poröse Struktur stellen die Fluidverbindung zwischen dem Vorratstank 17 und dem Strömungskanal 16 her.

Das Abdeckelement 28 dient quasi als Deckel 35, wobei der Deckel 35 wahlweise ein fester oder weicher Deckel 35 ist. Der Deckel 35 ist über der Öffnung 27 derart lösbar oder nicht lösbar angebracht, dass das granulare Material, also lose Körner 24 oder miteinander verbundene Körner 24, zuverlässig in der Position gehalten wird. Der Deckel 35 deckt mindestens die Öffnung 27 ab. In anderen Ausführungsformen umgibt der Deckel 35 jedoch den gesamten Hohlkörper 15 bzw. das Trägerelement 29 einschließlich der Öffnung 27 des Aufnahmeraums 26, wobei der Deckel 35 mindestens abschnittsweise flüssigkeitspermeabel, z.B. durch eine Perforation ausgebildet ist. Der Deckel 35 ist nicht auf eine klassische Deckelform beschränkt, sondern kann beliebige Formen aufweisen. Wie erwähnt kann der Deckel 35 auch aus unterschiedlichen Materialien und Materialkombinationen bestehen.

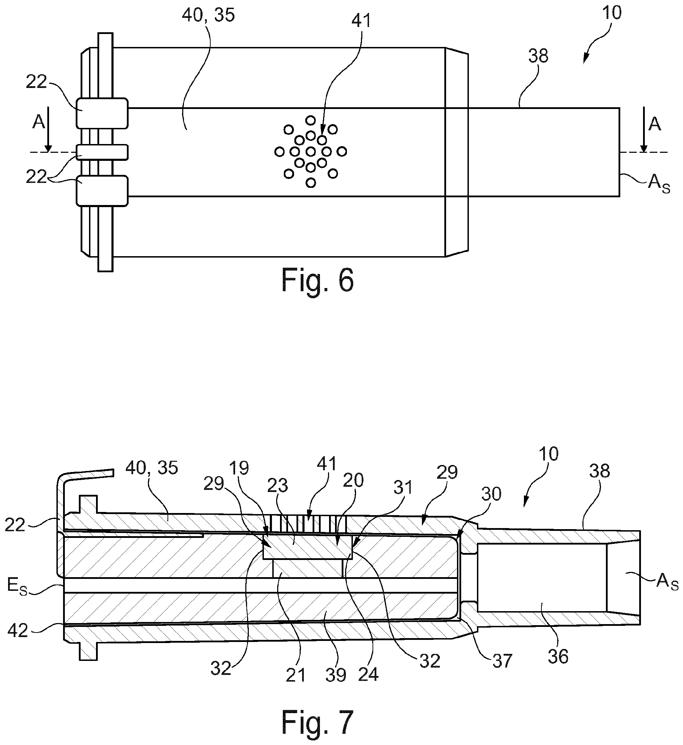

Ein fester Deckel 35 z.B. aus Kunststoff ist den Figuren 6 und 7 zu entnehmen. In dieser Ausführungsform ist das Trägerelement 29 rohrförmig mit der Durchgangsöffnung 30 ausgebildet. Innerhalb der Durchgangsöffnung 30 sind zwei miteinander verbundene Kammern 36, 37 ausgebildet. Die eine Kammer 36 dient als Mundstück 38. In der zweiten Kammer 37 ist ein Einsatz 39 angeordnet, der die Verdampfereinheit 19 mit dem Heizorgan 21 und dem Dochtorgan 20 trägt. Der Einsatz 39 weist die Ausnehmung 31 auf, durch die die Seitenwände 32 gebildet sind, um den Aufnahmeraum 26 zu definieren. Die Verdampfereinheit 19 und damit auch das Dochtorgan 20 sind durch den Einsatz 39 und die Innenseite einer Wandung 40 des Trägerelementes 29 in Position gehalten. Die Wandung 40 des Trägerelementes 29 bildet somit den harten Deckel 35. Der Einsatz 39 weist des Weiteren eine Durchgangsöffnung 42 auf, die mit der ersten Kammer 36 zur Bildung des Strömungskanals 16 in Wirkverbindung steht. Das Trägerelement 29 weist in der Wandung 40 in dem Bereich, an dem das Dochtorgan 20 an die Innenseite der Wandung 40 des rohrförmigen Trägerelementes 29 stößt, eine Perforation 41 auf, die eine Flüssigkeitskopplung zum Vorratstank 17 sicherstellt. Der Vorratstank 17 wird entsprechend zwischen der Gehäusewand 34 des Gehäuses 33 und der Wandung 40 des Trägerelementes 29 gebildet.

Die Deckel 35 können auf unterschiedliche Weise gebildet sein. Neben den beschriebenen Varianten besteht beispielsweise die Möglichkeit, Deckel 35 aus einer aushärtbaren Materiallage, z.B. einem aushärtbaren Netz, oder aus einem (Polyetheretherketon-)PEEK-Sieb, das mit dem Trägerelement 29 verschweißt wird, zu bilden, um die Öffnung 27 des Aufnahmeraums 26 zu schließen.

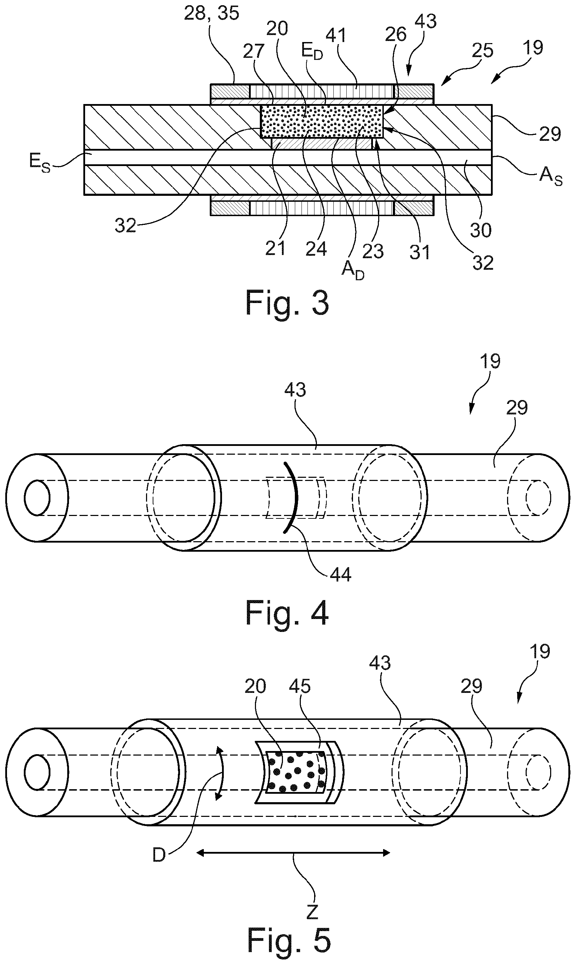

Ein weicher Deckel 35 ist beispielhaft in den Figuren 3 bis 5 dargestellt. Der Deckel 35 ist eine gegenüber dem vorzugsweise zylindrisch ausgebildeten Hohlkörper 15 als Trägerelement 29 längsaxial oder rotatorisch verschiebbare Hülse 43, die mindestens abschnittsweise flüssigkeitspermeabel ausgebildet ist. Mit anderen Worten kann die Hülse 43 zum Abdecken des Dochtorgans 20 und Halten desselben in der Position um die Längsachse gedreht oder entlang dieser verschoben werden. Der Hohlkörper 15 kann aber auch andere Formen aufweisen und z.B. oval geformt sein. Die Hülse 43, die auch aus einem harten Material bestehen kann, ist vorzugsweise flexibel ausgebildet und ein Kunststoff- oder Silikonring mit flüssigkeitspermeablen Abschnitten, die eine Perforation 41 bilden können. In der Figur 3 ist beispielsweise ein rohrförmiges Trägerelement 29 mit einer durchgängigen Durchgangsöffnung 30 dargestellt, das in der Ausnehmung 31 die zweiteilige Verdampfereinheit 19 trägt. Gerade eine elastische Hülse 43 kann sich an unterschiedliche Formen/Konturen des Hohlkörpers 15 anpassen.

Die Verdampfereinheit 19 ist durch die elastische Hülse 43, die mindestens im Bereich des Dochtorgans 20 flüssigkeitspermeabel ausgebildet ist, in der Ausnehmung 31 gehalten. In den Figuren 4 und 5 ist jeweils ein rohrförmiges Trägerelement 29 dargestellt, bei dem die Verdampfereinheit 19 ebenfalls durch eine elastische Hülse 43 in Position gehalten wird. In der Figur 4 deckt die Hülse 43 das Dochtorgan 20 ab. Die Hülse 43 weist jedoch einen Schlitz 44 auf, der sich durch Zug (siehe Pfeil Z) an der Hülse 43 zu einem Fenster 45 (siehe Figur 5) öffnet, um das Dochtorgan 20 freizugeben. Anstelle des Schlitzes 44 kann grundsätzlich das Fenster 45 vorgesehen sein, dass durch Drehen (siehe Pfeil D) oder Schieben der Hülse 43 aus einer Position, in der die Hülse 43 das Dochtorgan 20 abdeckt, in eine Position, in der das Fenster 45 über dem Dochtorgan 20 liegt, bewegbar ist. Die Hülse 43, die auch ein Schlauch sein kann, ist vorzugsweise aus Silikon gebildet. In anderen Ausführungsformen weist der Deckel 35 einen gewickelten Vliesstreifen auf oder ist aus einem gewickelten Vliesstreifen gebildet. Weiterhin kann der Deckel 35 auch ein Pflaster, ein Netz, ein Schrumpfschlauch oder dergleichen sein.

Der Deckel 35, also das Element, das das granulare Dochtorgan 20, z.B. aus Sand und/oder Graphit gebildet, in Position hält, kann auch aus einer ringförmigen Schaumstoffscheibe mit einer Durchgangsbohrung gebildet sein. Ein solcher so genannter "Schaumstoff-Donut" kann mit der Durchgangsbohrung über ein rohrförmiges Trägerelement 29 gestülpt sein, derart, dass die Öffnung 27 des die Verdampfereinheit 19 und damit das Dochtorgan 20 umgebenden Aufnahmeraums 26 in dem Trägerelement 29 durch den Schaumstoffring abgedeckt ist, so dass das Dochtorgan 20 in seiner Position gehalten wird. Eine Offenporigkeit des Schaumstoffrings stellt die Fluidverbindung zum Vorratstank 17 bereit.

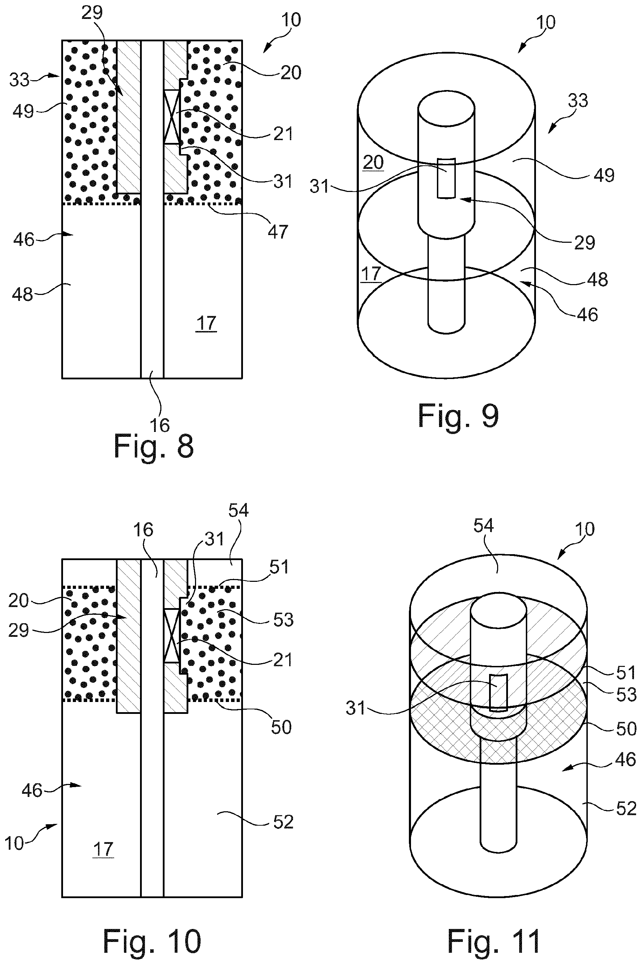

Die Figuren 8 bis 11 zeigen weitere Ausführungsformen möglicher Verdampferkartuschen 10. Bei diesen Ausführungsformen ist ein zylinderförmiges Gehäuse 33, das auch jede andere Form aufweisen kann, zur Bildung eines Innenraums 46 vorgesehen, in dem als Strömungskanal 16 ein durchgängiger Schlot vorgesehen ist. Das Trägerelement 29 erstreckt sich über einen Teil des Strömungskanals 16. In dem Trägerelement 29 ist die Ausnehmung 31 ausgebildet, in dem das Heizorgan 21 angeordnet ist. In der Variante der Figuren 8 und 9 teilt eine Struktur 47, vorzugsweise ein Gitterstruktur, den Innenraum 46 in zwei Bereiche 48, 49, wobei ein Bereich 48 als Vorratstank 17 für die Flüssigkeit und ein Bereich 49 zur Aufnahme des granularen Dochtorgans 20 dient, der der besseren Übersicht halber in den Figuren 8 und 10 nur angedeutet und in den Figuren 9 und 11 nicht explizit dargestellt ist. Anders ausgedrückt ist der Bereich 49 vollständig mit den granularen Körnern 24 des Dochtorgans 20, z.B. mit Sand ausgefüllt. Der Bereich 49 kann aber z.B. auch mit dem zuvor beschriebenen Schaumstoffring ausgefüllt sein. Die Struktur 47 ist unterhalb des Trägerelementes 29 angeordnet, derart, dass das Dochtorgan 20 das Trägerelement 29 vollständig umgibt und entsprechend auch das Heizorgan 21 abdeckt. Das Befestigungsmittel 25 mechanischer Art wird entsprechend durch die Gehäusewand 34 des Gehäuses 33, das Trägerelement 29, das Heizorgan 21 sowie die Struktur 47 gebildet, die zusammen das Dochtorgan 20, hier z.B. Sandkörner, in Position vor dem Heizorgan 21 halten. Die Struktur 47 stellt die Fluidverbindung zum Vorratstank 17 sicher.

In der Variante der Figuren 10 und 11 sind zwei Strukturen 50 und 51, vorzugsweise Gitterstrukturen, vorgesehen, die den Innenraum 46 in drei Bereiche 52, 53, 54 teilen. Die beiden Strukturen 50, 51 sind oberhalb und unterhalb der Ausnehmung 31 mit dem Heizorgan 21 angeordnet, derart, dass der mittlere Bereich 53, der zur Aufnahme des (nur angedeuteten bzw. nicht explizit dargestellten) Dochtorgans 20 dient, das Trägerelement 29 mindestens im Bereich des Heizorganes 21 abdeckt. In den anderen Bereichen 52, 54 kann Flüssigkeit bevorratet werden. Das Befestigungsmittel 25 mechanischer Art wird durch die Gehäusewand 34 des Gehäuses 33, das Trägerelement 29, das Heizorgan 21 sowie die Strukturen 50, 51 gebildet, die zusammen das granulare Dochtorgan 20, hier z.B. Sandkörner, in Position vor dem Heizorgan 21 halten. Die Strukturen 50, 51 stellen die Fluidverbindung zum Vorratstank 17 sicher. Anstelle von Sandkörnern oder anderen granularen Materialien kann zwischen den Strukturen 50, 51 z.B. auch ein offenporiger Schaumstoff angeordnet sein.

Alternativ oder kumulativ kann das Befestigungsmittel 25 physikalischer und/oder chemischer Art sein, derart, dass die Körner 24 durch Schmelz- und/oder Aushärteprozesse in Position gehalten werden. Die Körner 24 können z.B. mit dem Hohlkörper 15 bzw. dem Trägerelement 29 oder Teilen davon verschmolzen sein. Aus miteinander verschmolzenen Körnern 24 des Dochtorgans 20 kann insbesondere in Verbindung mit dem Heizorgan 21 eine Art Käfig gebildet sein, in dem lose Körner 24 des Dochtorgans 20 in Position gehalten werden. Es können optional auch Körner 24 zur Bildung eines Deckels 35 miteinander verschmolzen sein. Anstelle der Körner 24 kann zur Bildung eines Aufnahmeraums 26 und/oder eines Rückhalteelementes expandierbares Schaummaterial eingesetzt werden, das in der Position, in der es die Verdampfereinheit 19 umgibt oder abdeckt, ausgehärtet wird, um einen Deckel 35 zu schaffen, mit dem das Dochtorgan 20 in Position gehalten wird.

In weiteren, nicht explizit dargestellten Ausführungsformen ist das Befestigungsmittel 25 für das granulare Dochtorgan 20 alternativ oder kumulativ magnetischer Art, derart, dass die Körner 24 mindestens teilweise magnetisch ausgebildet sind und Magnete, die im Bereich des Dochtorgans 20 angeordnet sind, die Körner 24 in Position halten. Optional ist das Befestigungsmittel 25 alternativ oder kumulativ elektrostatischer Art, derart, dass die Körner 24 mindestens teilweise elektrisch geladen sind und elektrische Felder, die im Bereich des Dochtorgans 20 vorhanden sind, die Körner in Position halten.

Die Befestigungsmittel 25 sind ganz oder teilweise wahlweise für eine dauerhafte oder eine temporäre Befestigung ausgebildet und eingerichtet. Vorzugsweise sind Teile des Befestigungsmittels 25 für eine dauerhafte Befestigung ausgebildet und eingerichtet, beispielsweise durch konstruktive Komponenten in Form von Seitenwänden 32, Heizorgan 21, Abdeckelementen 28/Deckeln 35 oder dergleichen, die das Dochtorgan 20 in montiertem Zustand bis zum Ende der Lebensdauer der Verdampferkartusche 10 in Position halten. Andere Teile des Befestigungsmittels 25, die insbesondere das Einbringen des Dochtorgans 20 erleichtern, sind dem Dochtorgan 20 selbst immanent, beispielsweise schockgefrorene Flüssigkeit-Granulat-Pasten, die das Dochtorgan 20 bis zum finalen Einbau in die Verdampferkartusche 10 in Position halten bzw. zusammenhalten.

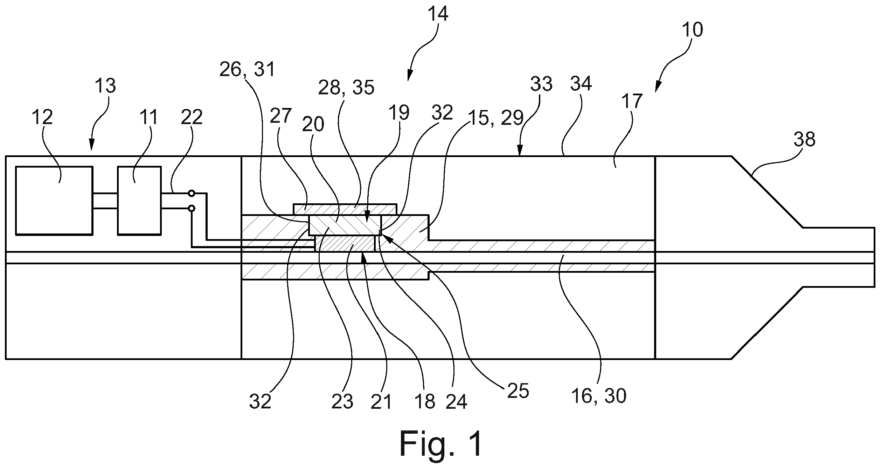

Das Funktionsprinzip des erfindungsgemäßen Inhalators 14 wird beispielhaft anhand einer E-Zigarette als Inhalator 14 insbesondere mit Bezug auf die Figur 1 beschrieben. Der Inhalator 14 ist zum Inhalieren von mit Wirkstoffen angereichertem Dampf ausgebildet und eingerichtet und umfasst einen mindestens eine elektronische Steuereinheit 11 und eine Energiequelle 12 umfassenden Kartuschenträger 13 sowie eine Verdampferkartusche 10, die sich erfindungsgemäß dadurch auszeichnet, dass sie nach einem oder mehreren der Ansprüche 1 bis 15 ausgebildet und eingerichtet ist.

Eine konsumierende Person saugt z.B. an einem Mundstück 38 des Inhalators 14, der aus dem Kartuschenträger 13 und der Verdampferkartusche 10 gebildet ist, wobei sich in dem Vorratstank 17 der Verdampferkartusche 10 eine Flüssigkeit befindet, die beispielsweise Glycerin, Propylenglycol und ggf. weitere Wirkstoffe und/oder Geschmacksstoffe enthält. Durch das Saugen wird in dem Strömungskanal 16 ein Unterdruck erzeugt, der seinerseits z.B. über einen nicht dargestellten Sensor die Steuereinheit 11 aktiviert. Die Steuereinheit 11 steuert das Heizorgan 21, das von der Energiequelle 12 mit Energie versorgt wird. Flüssigkeit aus dem Vorratstank 17 wird mittels des Dochtorgans 20 mindestens initial kapillar durch die Mikrokanäle 23 aus dem Vorratstank 17 in Richtung des Heizorganes 21 transportiert. Am bzw. im erwärmten Heizorgan 21 wird die Flüssigkeit zu Dampf umgewandelt, wobei das Heizorgan 21 die Flüssigkeit bzw. den daraus gebildeten Dampf aufgrund der flüssigkeits- und dampfpermeablen Struktur in Richtung des Strömungskanals 16 transportiert und an diesen abgibt. Der Dampf vermischt sich in dem Strömungskanal 16 mit dem Luftstrom und wird von der konsumierenden Person angesaugt und inhaliert.

## Patentansprüche

1. Verdampferkartusche (10) als Bestandteil eines Inhalators, umfassend einen Hohlkörper (15) mit einem durchgängigen Strömungskanal (16) sowie einen Vorratstank (17) zum Bevorraten von Flüssigkeit, wobei der Vorratstank (17) mindestens eine Zugangsöffnung (18) zum Strömungskanal (16) aufweist und im Bereich jeder Zugangsöffnung (18) eine sich über die gesamte Zugangsöffnung (18) erstreckende Verdampfereinheit (19) angeordnet ist, die ein Dochtorgan (20) und ein Heizorgan (21) aufweist, wobei die Verdampfereinheit (19) flüssigkeitspermeabel ausgebildet ist, derart, dass Flüssigkeit mindestens initial kapillar aus dem Vorratstank (17) durch die Verdampfereinheit (19) in Richtung des Strömungskanals (16) förderbar ist, **dadurch gekennzeichnet, dass** das Dochtorgan (20) aus einer Vielzahl granulatartiger, loser Körner (24) gebildet ist, die aufgrund ihrer Schüttung und/oder Ausbildung Mikrokanäle (23) bilden, die sich zur Herstellung einer Fluidverbindung zwischen dem Vorratstank (17) und dem Strömungskanal (16) durchgängig von einer Eintrittsseite E_{D} des Dochtorgans (20) zu einer Austrittsseite A_{D} des Dochtorgans (20) erstrecken, wobei ein zusätzliches Befestigungsmittel (25) vorgesehen ist, das ausgebildet und eingerichtet ist, das granulare Dochtorgan (20) in einer die Fluidverbindung aufrechterhaltenden Weise im Bereich der Zugangsöffnung (18) in Position zu halten.

2. Verdampferkartusche (10) nach Anspruch 1, **dadurch gekennzeichnet, dass** das Befestigungsmittel (25) mechanischer Art ist, derart, dass aus Komponenten der Verdampferkartusche (10) oder Teilen davon ein allseitig umschlossener Aufnahmeraum (26) mit einer Öffnung (27) zum Einbringen des granularen Dochtorgans (20) gebildet und die Öffnung (27) mit einem Abdeckelement (28) verschlossen ist, wobei der Aufnahmeraum (26) mindestens abschnittsweise flüssigkeitspermeabel ausgebildet ist.

3. Verdampferkartusche (10) nach Anspruch 2, **dadurch gekennzeichnet, dass** die Verdampferkartusche (10) ein Trägerelement (29) umfasst, das den Hohlkörper (15) bildet und zum einen eine Durchgangsöffnung (30) zur Bildung des Strömungskanals (16) aufweist und zum anderen eine Ausnehmung (31) zur Aufnahme des Dochtorgans (20) aufweist, wobei die Ausnehmung (31) die Seitenwände (32) des Aufnahmeraums (26) bildet und die Ausnehmung (31) zum Vorratstank (17) hin und zum Strömungskanal (16) hin jeweils durch eine flüssigkeitspermeable Struktur begrenzt ist.

4. Verdampferkartusche (10) nach Anspruch 2 oder 3, **dadurch gekennzeichnet, dass** die Körner (24) des granularen Dochtorgans (20) durch ein Sieb in Position gehalten werden, derart, dass das Sieb zusammen mit den den Aufnahmeraum (26) begrenzenden Seitenwänden (32) und einem separat zum Dochtorgan (20) ausgebildeten Heizorgan (21) das Volumen des Dochtorgans (20) umschließt.

5. Verdampferkartusche (10) nach einem oder mehreren der Ansprüche 2 bis 4, **dadurch gekennzeichnet, dass** das Abdeckelement (28) wahlweise ein fester oder weicher Deckel (35) ist.

6. Verdampferkartusche (10) nach Anspruch 5, **dadurch gekennzeichnet, dass** der Deckel (35) mindestens die Öffnung (27) abdeckt, vorzugsweise jedoch den gesamten Hohlkörper (15) einschließlich der Öffnung (27) des Aufnahmeraums (26) umgibt, wobei der Deckel (35) mindestens abschnittsweise flüssigkeitspermeabel ausgebildet ist.

7. Verdampferkartusche (10) nach Anspruch 5 oder 6, **dadurch gekennzeichnet, dass** der Deckel (35) eine gegenüber dem Hohlkörper (15) längsaxial oder rotatorisch verschiebbare Hülse (43) ist, die mindestens abschnittsweise flüssigkeitspermeabel ausgebildet ist.

8. Verdampferkartusche (10) nach Anspruch 5 oder 6, **dadurch gekennzeichnet, dass** der Deckel (35) einen gewickelten Vliesstreifen aufweist oder aus einem gewickelten Vliesstreifen gebildet ist.

9. Verdampferkartusche (10) nach einem oder mehreren der Ansprüche 1 bis 8, **dadurch gekennzeichnet, dass** das Befestigungsmittel (25) magnetischer Art ist, derart, dass die Körner (24) mindestens teilweise magnetisch ausgebildet sind und Magnete, die im Bereich des Dochtorgans (20) angeordnet sind, die Körner (24) in Position halten.

10. Verdampferkartusche (10) nach einem oder mehreren der Ansprüche 1 bis 9, **dadurch gekennzeichnet, dass** das Befestigungsmittel (25) elektrostatischer Art ist, derart, dass die Körner (24) mindestens teilweise elektrisch geladen sind und elektrische Felder, die im Bereich des Dochtorgans (20) vorhanden sind, die Körner (24) in Position halten.

11. Inhalator (14), ausgebildet und eingerichtet zum Inhalieren von mit Wirkstoffen angereichertem Dampf, umfassend einen mindestens eine elektronische Steuereinheit (11) und eine Energiequelle (12) umfassenden Kartuschenträger (13) sowie eine Verdampferkartusche (10), **dadurch gekennzeichnet, dass** die Verdampferkartusche (10) nach einem oder mehreren der Ansprüche 1 bis 10 ausgebildet und eingerichtet ist.

## Claims

1. Vaporizer cartridge (10) as a component of an inhaler, comprising a hollow body (15) with a continuous flow channel (16) as well as a storage tank (17) for storing liquid, wherein the storage tank (17) has at least one access opening (18) to the flow channel (16) and a vaporizer unit (19) extending over the entire access opening (18) is arranged in the region of each access opening (18), which vaporizer unit (19) has a wick member (20) and a heating member (21), wherein the vaporizer unit (19) is formed to be liquid-permeable in such a manner that liquid can be conveyed at least initially in a capillary manner from the storage tank (17) through the vaporizer unit (19) in the direction of the flow channel (16), **characterised in that** the wick member (20) is formed from a plurality of granular loose grains (24) which, as a result of their fill and/or formation, form microchannels (23) which extend, for the establishment of a fluid connection between the storage tank (17) and the flow channel (16), continuously from an entry side E_{D} of the wick member (20) to an exit side A_{D} of the wick member (20), wherein an additional securing means (25) is provided which is configured and adapted to hold the granular wick member (20) in position in the region of the access opening (18) in a manner which maintains the fluid connection.

2. Vaporizer cartridge (10) according to Claim 1, **characterised in that** the securing means (25) is of a mechanical type such that a receiving chamber (26) enclosed on all sides with an opening (27) for the introduction of the granular wick member (20) is formed from components of the vaporizer cartridge (10) or parts thereof and the opening (27) is closed with a cover element (28), wherein the receiving chamber (26) is formed to be liquid-permeable at least in sections.

3. Vaporizer cartridge (10) according to Claim 2, **characterised in that** the vaporizer cartridge (10) comprises a carrier element (29) which forms the hollow body (15) and on the one hand has a through-opening (30) for forming the flow channel (16) and on the other hand has a recess (31) for receiving the wick member (20), wherein the recess (31) forms the side walls (32) of the receiving chamber (26) and the recess (31) towards the storage tank (17) and towards the flow channel (16) is delimited in each case by a liquid-permeable structure.

4. Vaporizer cartridge (10) according to Claim 2 or 3, **characterised in that** the grains (24) of the granular wick member (20) are held in position by a sieve in such a manner that the sieve, together with the side walls (32) delimiting the receiving chamber (26) and a heating member (21) formed separately from the wick member (20), encloses the volume of the wick member (20).

5. Vaporizer cartridge (10) according to one or more of Claims 2 to 4, **characterised in that** the cover element (28) is optionally a solid or soft lid (35).

6. Vaporizer cartridge (10) according to Claim 5, **characterised in that** the lid (35) covers at least the opening (27), preferably, however, the entire hollow body (15) including the opening (27) of the receiving chamber (26), wherein the lid (35) is formed to be liquid-permeable at least in sections.

7. Vaporizer cartridge (10) according to Claim 5 or 6, **characterised in that** the lid (35) is a sleeve (43) which is displaceable in a longitudinally axial or rotational manner with respect to the hollow body (15) and which is formed to be liquid-permeable at least in sections.

8. Vaporizer cartridge (10) according to Claim 5 or 6, **characterised in that** the lid (35) has a wound fleece strip or is formed from a wound fleece strip.

9. Vaporizer cartridge (10) according to one or more of Claims 1 to 8, **characterised in that** the securing means (25) is of a magnetic type such that the grains (24) are formed to be at least partially magnetic and magnets which are arranged in the region of the wick member (20) hold the grains (24) in position.

10. Vaporizer cartridge (10) according to one or more of Claims 1 to 9, **characterised in that** the securing means (25) is of an electrostatic type such that the grains (24) are at least partially electrically charged and electric fields which are present in the region of the wick member (20) hold the grains (24) in position.

11. Inhaler (14), configured and adapted for the inhalation of vapour enriched with active ingredients, comprising a cartridge carrier (13) at least comprising an electronic control unit (11) and an energy source (12) as well as a vaporizer cartridge (10), c h a r act e r is e d in that the vaporizer cartridge (10) is configured and adapted according to one or more of Claims 1 to 10.

## Revendications

1. Cartouche de vaporisation (10) en tant que constituant d'un inhalateur, comprenant un corps creux (15) avec un canal d'écoulement continu (16) ainsi qu'un réservoir de stockage (17) pour le stockage de liquide, le réservoir de stockage (17) présentant au moins une ouverture d'accès (18) au canal d'écoulement (16) et une unité de vaporisation (19) s'étendant sur toute l'ouverture d'accès (18) étant agencée dans la zone de chaque ouverture d'accès (18), qui présente un organe formant mèche (20) et un organe de chauffage (21), l'unité de vaporisation (19) étant configurée de manière à être perméable aux liquides, de telle sorte que du liquide peut être transporté au moins initialement par capillarité depuis le réservoir de stockage (17) à travers l'unité de vaporisation (19) en direction du canal d'écoulement (16), **caractérisée en ce que** l'organe formant mèche (20) est formé d'une pluralité de grains lâches (24) de type granulat qui, en raison de leur remplissage et/ou de leur configuration, forment des micro-canaux (23) qui s'étendent en continu d'un côté d'entrée E_{D} de l'organe formant mèche (20) à un côté de sortie A_{D} de l'organe formant mèche (20) pour établir une communication fluidique entre le réservoir de stockage (17) et le canal d'écoulement (16), il étant prévu un moyen de fixation supplémentaire (25) qui est configuré et adapté pour maintenir l'organe formant mèche granulaire (20) en position dans la zone de l'ouverture d'accès (18) d'une manière qui maintient la communication fluidique.

2. Cartouche de vaporisation (10) selon la revendication 1, **caractérisée en ce que** le moyen de fixation (25) est de type mécanique, de telle sorte qu'à partir de composants de la cartouche de vaporisation (10) ou de parties de celle-ci, un espace de réception (26) entouré de tous côtés est formé avec une ouverture (27) pour l'introduction de l'organe formant mèche granulaire (20) et l'ouverture (27) est fermée par un élément de recouvrement (28), l'espace de réception (26) étant configuré au moins par sections de manière à être perméable aux liquides.

3. Cartouche de vaporisation (10) selon la revendication 2, **caractérisée en ce que** la cartouche de vaporisation (10) comprend un élément de support (29) qui forme le corps creux (15) et qui présente d'une part une ouverture de passage (30) pour former le canal d'écoulement (16) et d'autre part un évidement (31) pour recevoir l'organe formant mèche (20), l'évidement (31) formant les parois latérales (32) de l'espace de réception (26) et l'évidement (31) étant délimité vers le réservoir de stockage (17) et vers le canal d'écoulement (16) respectivement par une structure perméable aux liquides.

4. Cartouche de vaporisation (10) selon la revendication 2 ou 3, **caractérisée en ce que** les grains (24) de l'organe formant mèche granulaire (20) sont maintenus en position par un tamis, de telle sorte que le tamis entoure le volume de l'organe formant mèche (20) conjointement avec les parois latérales (32) délimitant l'espace de réception (26) et un organe de chauffage (21) configuré séparément de l'organe formant mèche (20).

5. Cartouche de vaporisation (10) selon une ou plusieurs des revendications 2 à 4, **caractérisée en ce que** l'élément de recouvrement (28) est au choix un couvercle rigide ou souple (35).

6. Cartouche de vaporisation (10) selon la revendication 5, **caractérisée en ce que** le couvercle (35) recouvre au moins l'ouverture (27), mais entoure de préférence l'ensemble du corps creux (15), y compris l'ouverture (27) de l'espace de réception (26), le couvercle (35) étant configuré de manière à être perméable aux liquides au moins par sections.

7. Cartouche de vaporisation (10) selon la revendication 5 ou 6, **caractérisée en ce que** le couvercle (35) est un manchon (43) pouvant être déplacé par rapport au corps creux (15) dans le sens de l'axe longitudinal ou en rotation, qui est configuré de manière à être perméable aux liquides au moins par sections.

8. Cartouche de vaporisation (10) selon la revendication 5 ou 6, **caractérisée en ce que** le couvercle (35) présente une bande de non-tissé enroulée ou est formé d'une bande de non-tissé enroulée.

9. Cartouche de vaporisation (10) selon l'une ou plusieurs des revendications 1 à 8, **caractérisée en ce que** le moyen de fixation (25) est de type magnétique, de telle sorte que les grains (24) sont configurés au moins partiellement sous forme magnétique et des aimants agencés dans la zone de l'organe formant mèche (20) maintiennent les grains (24) en position.

10. Cartouche de vaporisation (10) selon une ou plusieurs des revendications 1 à 9, **caractérisée en ce que** le moyen de fixation (25) est de type électrostatique, de telle sorte que les grains (24) sont au moins partiellement chargés électriquement et des champs électriques présents dans la zone de l'organe formant mèche (20) maintiennent les grains (24) en position.

11. Inhalateur (14), configuré et adapté pour inhaler de la vapeur enrichie en principes actifs, comprenant un support de cartouche (13) qui comprend au moins une unité de commande électronique (11) et une source d'énergie (12), ainsi qu'une cartouche de vaporisation (10), **caractérisé en ce que** la cartouche de vaporisation (10) est configurée et adaptée selon une ou plusieurs des revendications 1 à 10.
